# EUROPEAN PATENT SPECIFICATION

(11) **EP 4 279 121 B1**
(45) Date of publication and mention of the grant of the patent: **24.07.2024**
(21) Application number: 22174480.8
(22) Date of filing: 20.05.2022
(51) Int. Cl.: A61N 1/365, A61N 1/375, A61N 1/368

(54) **AN IMPLANTABLE MEDICAL DEVICE CONFIGURED TO PROVIDE AN INTRA-CARDIAC PACING**
IMPLANTIERBARE MEDIZINISCHE VORRICHTUNG, DIE SO KONFIGURIERT IST, DASS SIE EINE INTRAKARDIALE STIMULATION BEREITSTELLT
DISPOSITIF MÉDICAL IMPLANTABLE CONÇU POUR FOURNIR UNE STIMULATION INTRACARDIAQUE

(43) Date of publication of application: 22.11.2023
(73) Proprietor: BIOTRONIK SE & Co. KG, 12359 Berlin (DE)
(72) Inventor: Swenson, Kurt, Dayton, 97114 (US); Taff, Brian M., Portland, 97217 (US); McMillan, Brad, Lake Oswego, 97035 (US); Freiberg, Karl-Heinz, Woodburn, 97071 (US); Miller, David, Lake Oswego, 97035 (US); Kraetschmer, Hannes, West Linn, 97068 (US); Muessig, Dirk, West Linn, 97068 (US); Midgett, Madeline Anne, Portland, 97213 (US); Whittington, R. Hollis, Portland, 97202 (US); Jones, Christopher, Oregon City, 97045 (US); Young, Daniel, Portland, 97202 (US)
(74) Representative: Biotronik Corporate Services SE

(56) References cited:
- EP-A1- 2 490 758
- EP-A1- 3 191 174
- EP-A1- 3 532 156

## Description

The instant invention generally relates to an implantable medical device for providing an intra-cardiac pacing, specifically a VDD pacing.

In an implantable medical device, e.g. in the shape of a leadless pacemaker device, it may be desirous to provide for a stimulation in a ventricle of the patient's heart, e.g. in the right ventricle, in synchrony with an atrial activity. For this, a ventricular pacing action shall take into account atrial sense signals to control the ventricular pacing based on atrial sense events indicative of atrial activity, for example in a so-called VDD pacing mode.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a capsule for implantation on cardiac tissue, in particular the right ventricular wall of the right ventricle. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

A leadless pacemaker may specifically be designed for implantation in the right ventricle and, in this case, during implant is placed e.g. along the mid-septal wall of the right ventricle. Ventricular pacing may, for example, be indicated in cases where a dysfunction at the AV node occurs, but the sinus node function remains intact and appropriate. In such a case, in particular, a so-called VDD pacing mode therapy may be desired, involving a ventricular pacing that occurs in accordance with tracked atrial events and hence requires a sensing of atrial activity to pace at the ventricle in coordination with intrinsic atrial contractions. VDD pacing is, in particular, motivated by patient hemodynamic benefits of atrioventricular (AV) synchrony and utilizes an appropriate sinus node function to trigger ventricular pacing, offering the potential to maximize ventricular preload, limit AV valve regurgitation, maintain low mean atrial pressure, and to, furthermore, regulate autonomic and neurohumoral reflexes.

VDD pacing generally requires a tracking of atrial events, wherein information about the intrinsic cardiac rate and the relative atrial and ventricular event phasing within each cardiac cycle proves necessary. For leadless pacemaker systems where a single device resides within the patient's right ventricle, an atrial event detection is inherently challenged compared to traditional multi-lead transvenous embodiments, as atrial signals are not directly measured by the leadless pacer through direct physical interfacing with the heart's contraction/conduction activities.

US 9,492,668 discloses a leadless pacing device configured for implantation in a ventricle of a heart of a patient. The leadless pacing device is configured to switch between an atrioventricular synchronous pacing mode and an asynchronous ventricular pacing mode in response to detection of one or more sensing events, which may be, for example, undersensing events.

US 9,724,519 discloses a leadless pacing device configured to switch from a sensing without pacing mode to a ventricular pacing mode in response to determining that no intrinsic ventricular activity was detected within a ventricular event detection window for at least one cardiac cycle. The ventricular pacing mode may be selected based on whether heart rate oversensing is detected in combination with a loss of conduction. In some examples, an atrioventricular synchronous pacing mode is selected in response to detecting loss of conduction and in response to determining that atrial oversensing is not detected. In addition, in some examples, an asynchronous ventricular pacing mode is selected in response to detecting both atrial oversensing and loss of conduction. EP2490758 A1 discloses an implantable medical device according to the preamble of claim 1.

The invention is defined in claim 1. Any methods disclosed hereinafter do not form part of the scope of the invention and are mentioned for illustrative purposes only.

It is an object to provide an implantable medical device and a method for operating an implantable medical device allowing, in particular, for a ventricular pacing with atrioventricular synchrony in case atrial events are detected, but ensuring a reliable pacing even when no atrial events can be detected.

In one aspect, an implantable medical device configured to provide for an intracardiac pacing comprises: an electrode arrangement configured to sense a cardiac sense signal; and a processing circuitry operatively connected to the electrode arrangement.

The processing circuitry is configured to start a first timer based on a ventricular sense event identified using the cardiac sense signal or based on a ventricular pace event; to open an atrial detection window based on a first timer count output by the first timer; to start a second timer in case an atrial sense event is identified using the cardiac sense signals after opening the atrial detection window; to identify a first timeout based on a comparison of a second timer count output by the second timer and a first pacing delay indicative of a delay after which a ventricular pace signal should be triggered following a prior atrial sense event; to identify a second timeout based on a comparison of the first timer count output by the first timer and a second pacing delay indicative of a delay after which a ventricular pace signal should be triggered following a prior ventricular sense event or ventricular pace event; to identify a third timeout based on a comparison of the first timer count output by the first timer and a basic rate interval indicative of a longest allowable interval without a ventricular sense event or ventricular pace event; and to trigger a ventricular pace signal based on an identification of at least one of the first timeout, the second timeout and the third timeout. The implantable medical device in particular may be a leadless implantable medical device, such as a leadless pacemaker device. The implantable medical device in particular may be configured for implantation into a ventricle of the patient's heart, for example the right ventricle, for providing for a stimulation within the ventricle of the patient's heart.

The implantable medical device comprises a sensing arrangement which, in particular, may comprise a pair of electrodes by means of which cardiac sense signals are sensed. The sensing arrangement herein, in particular, may be comprised of a single pair of electrodes, by means of which a cardiac sense signal is detected. Such a sensing arrangement with the pair of electrodes may also be called electrode arrangement. Based on the cardiac sense signal both atrial events and ventricular events are identified. The sensing arrangement in particular may be comprised of a tip electrode arranged on a tip of a housing, in particular on a distal tip of the housing, of the implantable medical device to be placed on intracardiac tissue and a return electrode, in particular a proximal return electrode, placed on the housing of the implantable medical device at a location at some distance from the tip of the implantable medical device.

Generally, when sensing atrial events in a situation in which the implantable medical device is implanted in the ventricle of the heart, signals caused by atrial events will be small and hence difficult to detect. Atrial event detection hence is practical only if it takes place in a specific atrial detection window in which other signal contributions, in particular relating to ventricular activity, are small. Atrial events herein can be assumed to fall into the atrial detection window in case atrial and ventricular activity is in synchronization. When atrial activity and ventricular activity are out of sync, the atrial signal will be continuously changing its phase position within the cardiac cycle and will not be guaranteed to be detectable within the atrial detection window.

For establishing a pacing that involves an atrial tracking, but ensures a reliable pacing even subject to unreliable atrial event detection, it herein is proposed to implement a timer model which makes use of different timers to allow for a detection of atrial events (if possible), but at the same time ensure that a pacing is administered by extrapolating atrial timing information when atrial event detection is temporarily absent.

To support this balance in accessing atrial tracking while also ensuring robust bradycardia symptom mitigation subject to challenged atrial detection, a first timer, also denoted as main timer, is started based on an identified ventricular sense event or a ventricular pace event. The start of the first timer hence indicates the start of a cardiac cycle.

Based on the first timer, the start of an atrial detection window is determined. E.g. by comparing a first timer count output by the first timer to an atrial detection window start time the atrial detection window is opened, and within the atrial detection window it is searched for an atrial sense event.

If, in the atrial detection window, an atrial sense event is identified, a second timer is started. The second timer hence measures the time following the detection of an atrial sense event within the atrial detection window.

Based on the two timers, different timeouts are determined, and based on the timeouts a ventricular pace signal is triggered.

Namely, a first timeout is identified based on a comparison of a second timer count output by the second timer and a first pacing delay indicative of a delay after which a ventricular pace signal should be triggered following a prior atrial sense event. If within a time span following a detected atrial sense event no ventricular sense event is identified, but if the first pacing delay is reached without detecting a ventricular sense event, it may be assumed that a ventricular pace signal should be triggered, and accordingly the first timeout is identified.

In addition, the processing circuitry is configured to identify a second timeout based on a comparison of the first timer count output by the first timer and a second pacing delay indicative of a delay after which a ventricular pace signal should be triggered following a prior ventricular sense event or ventricular pace event. Whereas the first timeout is identified based on the second timer, the second timeout is identified based on the first timer. If it is found that a time span after the prior ventricular event (which may be a ventricular sense event or a ventricular pace event) has lapsed which matches the second pacing delay, it is assumed that a ventricular pace signal should be triggered and hence the second timeout is identified. To be clear, if the atrial detection window has been started, the second timeout should never occur or should be deemed invalid. Hence, in particular in a scenario in which no atrial sense event is detected within the atrial detection window and hence the second timer is never started, by means of the second timeout it is identified that no ventricular sense event is identified within a suitable time window (as indicated by the second pacing delay) following the prior ventricular event.

Further in addition, the processing circuitry is configured to identify a third timeout based on a comparison of the first timer count output by the first timer and a basic rate interval indicative of a longest allowable interval without a ventricular sense event or ventricular pace event. The basic rate interval hence indicates the longest time span that the patient should experience without either a ventricular sense or pace event. The basic rate interval may be programmed by a clinician and defines a fallback in case neither the first timeout nor the second timeout is identified within the basic rate interval or in case no ventricular pace signal is triggered based on the first timeout or the second timeout (for example when using a so-called hysteresis mode as shall be detailed further below) within the basic rate interval.

Based on at least one of the first timeout, the second timeout and the third timeout the processing circuitry is configured to trigger a ventricular pace signal for causing a pacing action. The triggering of the ventricular pacing herein not necessarily takes place immediately upon identifying any one of the timeouts. In one embodiment, the processing circuitry is configured to trigger a ventricular pacing based on a further processing of one or multiple timeouts which have been identified based on the first timer and the second timer. The hysteresis delays may be added to either the main timer interval or the AV delay window.

In one embodiment, the processing circuitry is configured to set the atrial detection window start time based on an average cardiac cycle interval time.

Alternatively or in addition, the processing circuitry may be configured to set the first pacing delay based on an average cardiac cycle interval time.

Alternatively or in addition, the processing circuitry may be configured to set the second pacing delay based on an average cardiac cycle interval time.

In certain embodiments, hence, the atrial detection window start time, the first pacing delay and/or the second pacing delay are not static, but may adaptive in accordance with changes in the heart rate. The average cardiac cycle interval time may, for example, be determined based on a ventricular-ventricular interval, which may be measured using the first timer by measuring a timing between subsequent ventricular events. For example, the ventricular-ventricular interval indicative of the (intrinsic or paced) heart rate may be measured over a multiplicity of cardiac cycles and may be averaged, for example by employing a moving averaging filter, a weighted averaging filter or another averaging mechanism, in order to determine an average cardiac cycle interval time. Based on the average cardiac cycle interval time, then, the atrial detection window start time, the first pacing delay and/or the second pacing delay may be set in order to adapt the specific timing value based on the actual heart rate as indicated by the average cardiac cycle interval time.

The averaging for determining the average cardiac cycle interval time may for example take place using a moving averaging filter involving a predefined number of cardiac cycles, such as a number in between 2 to 16 cardiac cycles.

The atrial detection window start time, the first pacing delay and/or the second pacing delay may for example be set based on a lookup using the average cardiac cycle interval time as an input. For example, a lookup table may be defined in which the atrial detection window start time, the first pacing delay and/or the second pacing delay are correlated to different values of the average cardiac cycle interval time, such that based on the lookup table the atrial detection window start time, the first pacing delay and/or the second pacing delay may be set.

An average cardiac cycle interval time may be determined equally for assessing the atrial detection window start time, the first pacing delay and the second pacing delay. In another embodiment, the average cardiac cycle interval time may be determined differently for setting the atrial detection window start time, the first pacing delay and the second pacing delay. For example, for setting the second pacing delay a different number of cardiac cycles, for example only the most recent cardiac cycles, may be included in the averaging when calculating a value for the average cardiac cycle interval time in comparison to the calculation of the average cardiac cycle interval time for setting the atrial detection window start time and the first pacing delay.

The second pacing delay, also denoted as flywheel interval, indicates a delay after which a ventricular pace should be triggered following a prior ventricular sense event or ventricular pace event. The second pacing delay may for example be set according to an average of most recent ventricular-ventricular intervals, for example to be equal to the average of the most recent ventricular-ventricular intervals or to exceed the average of the most recent ventricular-ventricular intervals by a predefined factor.

Generally, in one embodiment, the processing circuitry is configured to determine the ventricular-ventricular interval based on the first timer count at the time of a ventricular sense event or a ventricular pace event and to compute the average cardiac cycle interval time based on the ventricular-ventricular interval. Hence, by continuously determining the length of the cardiac cycles and by averaging over a predefined number of successive cardiac cycles an average cardiac cycle interval time may be determined, and based on the average cardiac interval time the atrial detection window start time, the first pacing delay and/or the second pacing delay may be set, for example by using a lookup based on the average cardiac cycle interval time as an input.

In case the ventricular-ventricular interval cannot be determined for a threshold number of cardiac cycles, in particular in cases where no atrial sense events and ventricular sense events are available within successive cardiac cycles, the processing circuitry may be enabled to switch to a different source in order to determine the ventricular-ventricular interval. For example, the processing circuitry may be configured to determine the ventricular-ventricular interval based on a sense signal received with a sensing device other than said sensing arrangement in case no ventricular sense event is identified in a predefined number of cardiac cycles. The other sensing device may, for example, be a sensing device employing a different sensing technology, such as an accelerometer measuring motion signals. Hence, if, by using the sensing arrangement employing a pair of electrodes, no ventricular sense events can be detected and thus no value for the ventricular-ventricular interval can be determined for a predefined number of cardiac cycles, it may be switched to an alternate source in order to measure the ventricular-ventricular interval and to determine an average cardiac cycle interval time based on the alternate source.

In another embodiment, the average cardiac cycle interval time may be set based on a programmed resting rate, in case no ventricular or atrial sense events are available for a predefined number of cardiac cycles.

The rate of change of the heart rate, as indicated by the average cardiac cycle interval time, can be controlled by logic that restricts the maximum change per cardiac cycle, such that a smooth transition from the prior heart rate to the new heart rate is established.

In one embodiment, the processing circuitry is configured to identify the second timeout if the first timer count matches the second pacing delay and no atrial sense event is identified after opening the atrial detection window. The second timeout hence is only identified if no atrial sense event is detected. The identification of the second timeout hence is gated by the detection of the atrial sense event, such that the second timeout may only be reached if no atrial sense event is detected. If an atrial sense event is detected, the first timeout is given precedence, in that it is checked whether a ventricular sense event is detected within the time span defined by the first pacing delay following the detection of the atrial sense event and the first timeout is identified if no ventricular sense event is detected before the first pacing delay is reached.

In one embodiment, the processing circuitry is configured to reset the first timer and the second timer in case a ventricular sense event is identified based on the cardiac sense signal or in case a ventricular pace event is triggered. Hence, if a ventricular sense event is detected prior to reaching the first timeout and/or the second timeout, the first timer and the second timer (in case it has been started upon detection of an atrial sense event) are reset. Hence, in case a ventricular sense event is detected before reaching any of the timeouts, the timeouts are reset and the process is started anew for the new cardiac cycle following the detection of the ventricular sense event. To be clear, only the main timer may be reset and restarted subject to a sensed ventricular event. While the others may have been reset, they do not restart at the same time as the main timer reset. If however no ventricular sense event is detected, and based on any one of the timeouts a ventricular pace signal is triggered to cause a ventricular pace event, the first timer and the second timer likewise are reset, such that following the ventricular pace event the process is started anew for the new cardiac cycle following the ventricular pace event.

Generally, the ventricular pace signal may be triggered in case of occurrence of any one of the first timeout, the second timeout and the third timeout. So far the third timeout has been focused/linked to the basic rate. The third timeout (or the first, for that matter) may be limited by the upper tracking rate (UTR) in atrial tracking conditions and by the maximum sensor rate (MSR) in accelerometer-driven modes. If a timeout occurs, hence, a ventricular pace signal is triggered and accordingly a ventricular pace event is caused.

In particular, in scenarios in which, based on recent history of cardiac activity, a ventricular sense event is likely to occur, for example when recent intrinsic atrioventricular (AV) conductions has been present, it may be desirous to postpone a ventricular pace and to allow for an additional waiting time before triggering a ventricular pace signal. This is denoted as hysteresis mode, in which ventricular sensing shall be encouraged over ventricular pacing.

To implement a hysteresis mode, in one embodiment the processing circuitry is configured to start a third timer based on the identification of at least one of the first timeout and the second timeout and to identify a fourth timeout based on a comparison of a third timer count output by the third timer and a hysteresis delay indicative of a delay after the first timeout or the second timeout. Hence, if the first timeout or the second timeout are identified, a ventricular pace signal is not immediately triggered, but another, third timer is started, and based on a timer count of the third timer the occurrence of a fourth timeout is monitored. In particular, the timer count of the third timer is compared to a hysteresis delay indicative of a delay after said first timeout or said second timeout at which a ventricular pace should occur. If the hysteresis delay is reached and hence the fourth timeout is identified, a ventricular pace signal may be triggered.

The hysteresis delay may for example be set based on an average cardiac cycle interval time, which as described before may be determined by averaging the ventricular-ventricular interval of successive cardiac cycles. The hysteresis delay herein may be determined based on a lookup using the average cardiac cycle interval time as an input. Alternatively, in a particularly efficient computational approach the hysteresis delay may be set by multiplying the first pacing delay with a factor, for example in a range between 0.2 and 0.8, for example 0.5.

The processing circuitry, in one embodiment, is configured to automatically switch to the hysteresis mode based on a processing of prior sense events. In particular, in one embodiment the processing circuitry is configured to set a hysteresis active signal based on at least one prior ventricular sense event and to start said third timer only if the hysteresis active signal is set. Hence, it is switched to the hysteresis mode if one or multiple ventricular sense events have been identified in prior cardiac cycles. Only if it is switched to the hysteresis mode and hence the hysteresis active signal is set, the third timer is started and hence the additional hysteresis delay is employed after identification of the first timeout or the second timeout.

The hysteresis active signal may for example be set based on logic that compares the ratio of ventricular senses to ventricular paces. This could be implemented for example as an up/down counter. For example, for each ventricular sense event the counter may count upwards, whereas on each ventricular pace event the counter may count downwards. In case the count may exceed a threshold, the hysteresis active signal may be set. An up-step size, a down-step size, an up-threshold and a down-threshold herein may be used to optimize a response time when AV conductions first start occurring after a prolonged pause as well as a response time when AV conductions stop after having been detected for some time.

If the hysteresis active signal is not set and hence the hysteresis mode is not used, a ventricular pace signal may be triggered for example immediately upon identifying the first timeout or the second timeout.

The hysteresis delay may, in particular, be employed after identification of the first timeout or the second timeout. In addition, the hysteresis delay mechanism may also be employed when identifying the third timeout.

In one embodiment, the processing circuitry is configured to reset the third timer in case a ventricular sense event is identified based on the cardiac sense signal or in case a ventricular pace event is triggered. Hence, if a ventricular sense event is identified prior to reaching the hysteresis delay or prior to reaching the termination of the hysteresis delay, and hence prior to identifying the fourth timeout, the third timer is reset. If no ventricular sense event is detected prior to reaching the hysteresis delay or the termination of the hysteresis delay, a ventricular pace signal is triggered and the third timer likewise is reset in order to start the process anew.

In another aspect, a method for operating an implantable medical device configured to provide for an intra-cardiac pacing comprises: sensing, using an electrode arrangement, a cardiac sense signal; starting, using a processing circuitry operatively connected to the electrode arrangement, a first timer based on a ventricular sense event identified using the cardiac sense signal or based a ventricular pace event; opening, using the processing circuitry, an atrial detection window based on a first timer count output by the first timer; starting, using the processing circuitry, a second timer in case an atrial sense event is identified using the cardiac sense signal after opening the atrial detection window; comparing, using the processing circuitry, a second timer count output by the second timer and a first pacing delay indicative of a delay after which a ventricular pace signal should be triggered following a prior atrial sense event to identify a first timeout; comparing, using the processing circuitry, the first timer count output by the first timer and a second pacing delay indicative of a delay after which a ventricular pace signal should be triggered following a prior ventricular sense event or ventricular pace event to identify a second timeout; comparing, using the processing circuitry, the first timer count output by the first timer and a basic rate interval indicative of a longest allowable interval without a ventricular sense event or ventricular pace event to identify a third timeout; and triggering, using the processing circuitry, a ventricular pace signal based on an identification of at least one of the first timeout, the second timeout and the third timeout.

The advantages and advantageous embodiments described above for the implantable medical device equally apply also to the method, such that it shall be referred to the above in this respect.

The various features and advantages of the present invention may be more readily understood with reference to the following detailed description and the embodiments shown in the drawings. Herein,
- Fig. 1: shows a schematic view of the human heart, with a leadless implantable medical device in the shape of a leadless pacemaker device implanted therein;
- Fig. 2: shows a schematic view of a leadless implantable medical device;
- Fig. 3: shows a schematic view of a processing circuitry of an embodiment of a leadless implantable medical device;
- Fig. 4A: shows a processing signal in the shape of an intra-cardiac electrogram (IEGM) processed by a first processing channel of the processing circuitry;
- Fig. 4B: shows a processing signal processed by a second processing channel of the processing circuitry;
- Fig. 5: shows a schematic circuit diagram of a timing stage of the processing circuitry configured to set a multiplicity of timeouts based on different delays;
- Fig. 6: shows an embodiment of a combining module for combining different timeouts to set a ventricular pace; and
- Fig. 7: shows another embodiment of a combining module for combining different timeouts to set a ventricular pace.

Subsequently, embodiments of the invention shall be described in detail with reference to the drawings. In the drawings, like reference numerals designate like structural elements.

It is to be noted that the embodiments are not limiting for the invention, but merely represent illustrative examples.

In the instant invention it is proposed to provide a leadless implantable medical device providing for an intra-cardiac function, in particular a ventricular pacing, specifically a so-called VDD pacing mode.

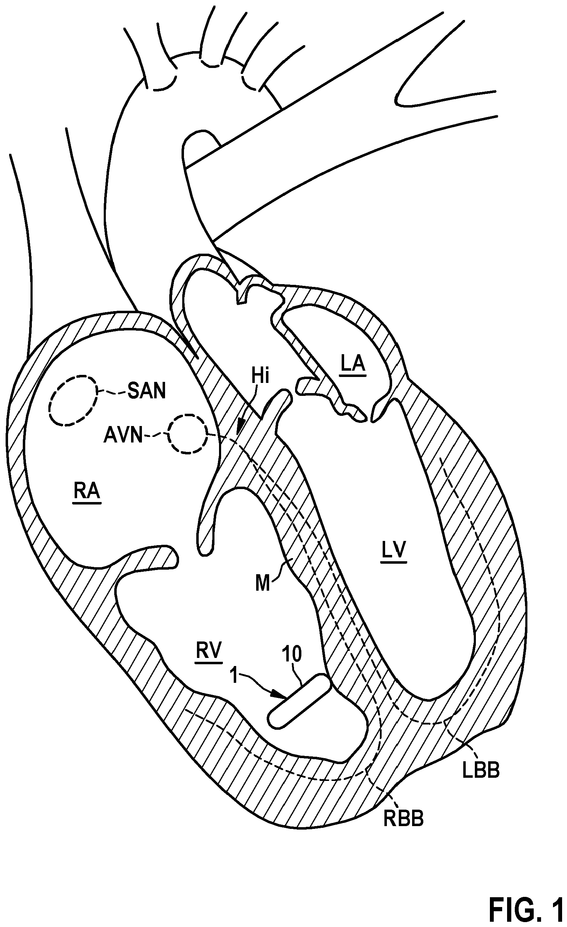

Fig. 1 shows, in a schematic drawing, the human heart comprising the right atrium RA, the right ventricle RV, the left atrium LA and the left ventricle LV, the so-called sinoatrial node SAN being located in the wall of the right atrium RA, the sinoatrial node SAN being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. From the atrioventricular node AVN the so-called HIS bundle Hi is extending, the HIS bundle Hi being comprised of heart muscle cells specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

In case of a block at the atrioventricular node AVN, the intrinsic electrical conduction system of the heart H may be disrupted, causing a potentially insufficient intrinsic stimulation of ventricular activity, i.e., insufficient or irregular contractions of the right and/or left ventricle RV, LV. In such a case, a pacing of ventricular activity by means of a pacemaker device may be indicated, such pacemaker device stimulating ventricular activity by injecting stimulation energy into intra-cardiac tissue, specifically myocardium M.

In one embodiment, a leadless implantable medical device 1 in the shape of a leadless pacemaker device, as schematically indicated in Fig. 1, is provided for a ventricular pacing action, the leadless pacemaker device having a body 10 formed by the housing of the leadless pacemaker device.

Whereas common leadless implantable medical devices are designed to sense a ventricular activity by receiving electrical signals from the ventricle RV, LV they are placed in, it may be desirable to provide for a pacing action which achieves atrioventricular (AV) synchrony by providing a pacing in the ventricle in synchrony with an intrinsic atrial activity. For such pacing mode, also denoted as VDD pacing mode, it is required to sense atrial activity and identify atrial sense events relating to atrial contractions in order to base a ventricular pacing on such atrial sense events.

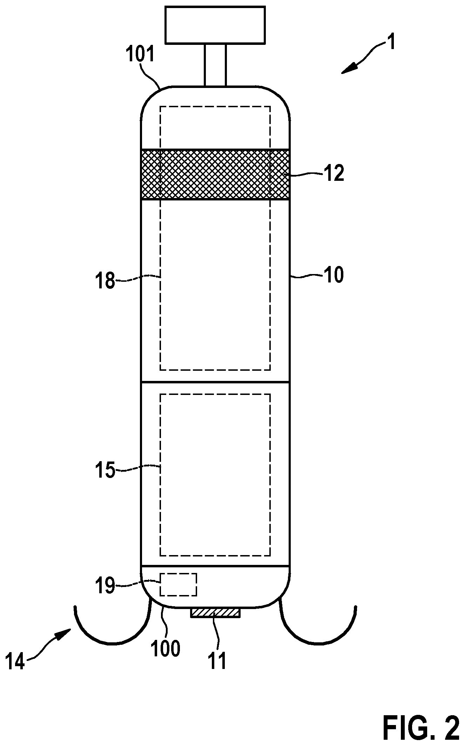

Referring now to Fig. 2, in one embodiment a leadless implantable medical device 1 in the shape of a leadless pacemaker device configured to provide for an intra-cardiac pacing, in particular in a VDD pacing mode, comprises a housing 10 enclosing electrical and electronic components for operating the leadless implantable medical device 1. In particular, enclosed within the housing 10 is a processing circuitry 15. In addition, electrical and electronic components such as a battery 18 are confined in the housing 10. The housing 10 provides for an encapsulation of components placed therein, the housing 10 having the shape of, e.g., a cylindrical capsule having a length of for example less than 4 centimeters, in particular less than 3 centimeters.

The leadless implantable medical device 1 is to be implanted immediately on or within intra-cardiac tissue M. For this implantation engagement, the leadless implantable medical device 1 comprises, in the region of a tip 100, a fixation device 14 for example in the shape of nitinol wires to engage with intra-cardiac tissue M for fixing the leadless implantable medical device 1 on the tissue in an implanted state.

The leadless implantable medical device 1 in the embodiment of Fig. 2 does not comprise leads, but senses signals relating to a cardiac activity by means of an electrode arrangement arranged on the housing 10 and also emits stimulation signals by means of such electrode arrangement. In the embodiment of Fig. 2, the leadless implantable medical device 1 comprises a pair of electrodes 11, 12 making up the electrode arrangement and serving to emit pace signals towards intra-cardiac tissue M for providing a pacing and to sense electrical signals indicative of a cardiac activity, in particular indicative of atrial and ventricular contractions. A first electrode 11 herein is denoted as pacing electrode, e.g. a cathode. The first electrode 11 is placed at the tip 100 of the housing 10 and is configured to contact with cardiac tissue M. A second electrode 12 is arranged on the housing 10 of the leadless implantable medical device 1 at some distance from the tip 100, e.g. at a location closer to an end 101 of the housing 10 opposite to the tip 100.

The electrodes 11, 12 are in operative connection with the processing circuitry 15. The processing circuitry 15 is configured to cause the pair of electrodes 11, 12 to emit a pace signal for providing a stimulation at the ventricle. The processing circuitry 15 furthermore is configured to process signals sensed via the pair of electrodes 11, 12 to provide for a sensing of cardiac activity, in particular, atrial and ventricular contractions.

In order to provide for a pacing in the ventricle in which the leadless implantable medical device 1 is placed, in particular, to enable a pacing in a VDD mode, a sensing of atrial activity is required to time a pacing in the ventricle to obtain atrioventricular (AV) synchrony. For this functionality, in addition to ventricular near field signals, atrial far-field signals must be sensed to identify atrial events.

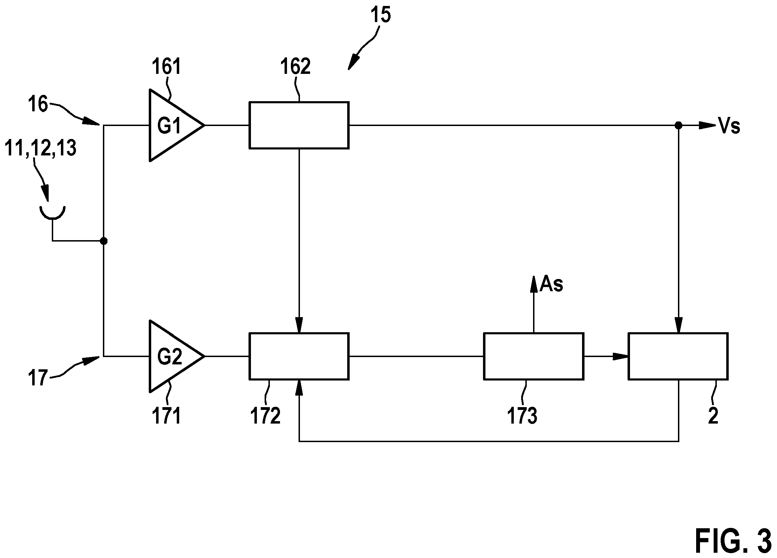

Referring now to Fig. 3, the processing circuitry 15 comprises, in one embodiment, two processing channels 16, 17 for processing signal portions relating to ventricular activity and atrial activity. Herein, typically, an intra-cardiac electrogram (IEGM) contains signal portions relating to ventricular activity (in particular a QRS wave) and atrial activity (in particular a P wave), signal portions relating to atrial activity however resulting from a far-field signal source and hence being far less pronounced and having a far smaller amplitude than signal portions relating to a ventricular activity in the near-field, i.e., arising in close proximity to the implanted leadless implantable medical device 1. For this reason, the two processing channels 16, 17 are associated with different gains G1, G2, a first processing channel 16 serving to process a sensed signal to identify ventricular sense events Vs at a rather low gain G1 and a second processing channel 17 being configured to process the sensed signal to identify atrial sense events As at a significantly higher gain G2.

To process sensed signals, both processing channels 16, 17 are connected to the electrode arrangement comprised of the electrodes 11, 12. Signal portions sensed via the pair of electrodes 11, 12 herein may be differentiated by employing a windowing scheme to allow for a signal detection of weak signal portions in the second processing channel 17.

The first processing channel 16 comprises a first amplification stage 161 having a gain G1 and, following the amplification stage 161, a detection stage 162 which is configured to identify ventricular sense events Vs.

The second processing channel 17 comprises a second amplification stage 171 having a second or different (higher than G1) gain G2, the second amplification stage 171 being followed by a windowing stage 172 and a second detection stage 173. The windowing stage 172 serves to identify an atrial detection window within the sensed signal, using a detected ventricular event Vs fed to the windowing stage 172 by the detection stage 162 as an input. The detection stage 173 conducts a preprocessing of the sense signal and evaluates and analyzes the processed signal in order to identify atrial sense events As.

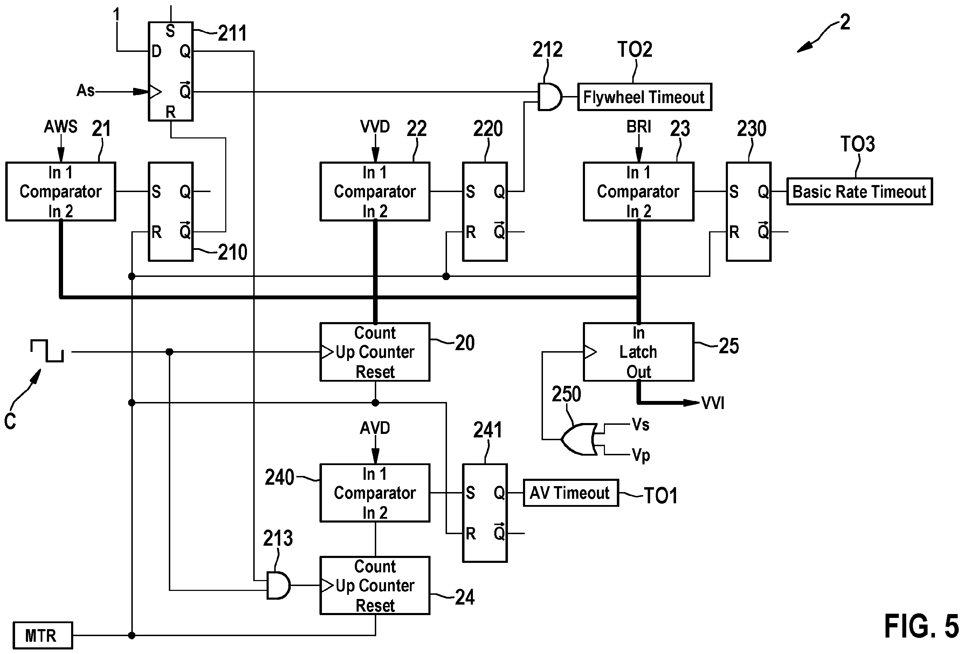

In addition, the processing circuitry 15 comprises a timing stage 2 which uses timing information sensed from the first processing channel 16 and the second processing channel 17 to provide for a pace timing, in particular a VDD timing for achieving an atrial-ventricular synchronous pacing, as shall be explained in more detail below with reference to Figs. 5 to 7.

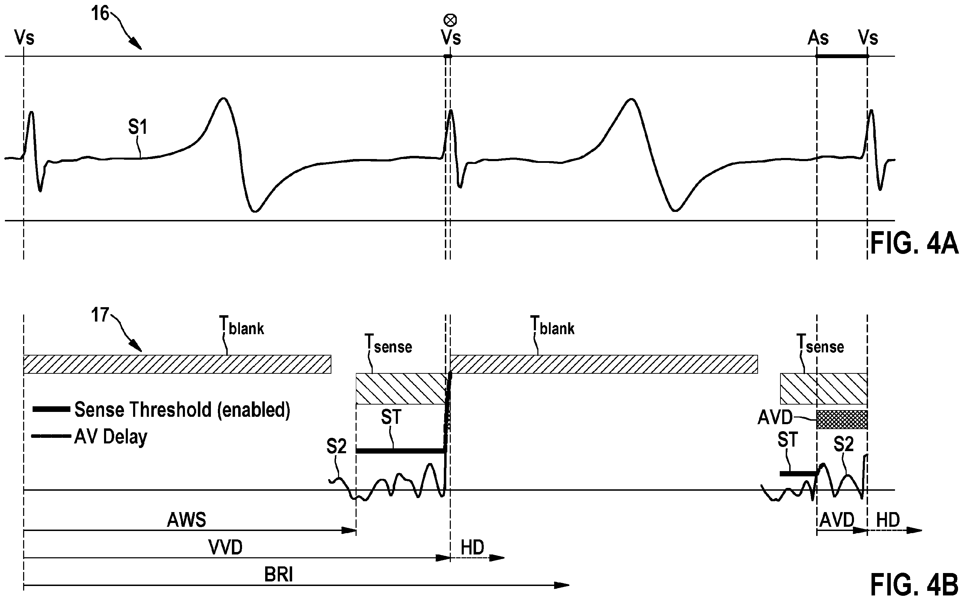

Figs. 4A and 4B show examples of signals S1, S2 as processed in the different processing channels 16, 17, Fig. 4A at the top showing a signal S1 as processed by the first processing channel 16 and Fig. 4B at the bottom showing a signal S2 as processed by the second processing channel 17. As a result of the processing, ventricular sense events Vs and atrial sense events As are identified and corresponding markers are output.

As apparent from Fig. 4B, the sensing of atrial sense events As uses a windowing scheme, employing in particular a blanking window T_{blank} for blanking out signal portions of the signal S2 potentially relating to ventricular activity. Namely, by means of the detection of ventricular sense events Vs in the first processing channel 16 an (expected) timing in between atrial sense events As and ventricular sense events Vs may be determined. According to such timing a start point and an end point of the blanking window T_{blank} may be set in the windowing stage 172, hence excluding signal portions from the processing in the second processing path 17 which do not relate to atrial activity. Strong ventricular signals in this way may be suppressed such that signal portions relating to a ventricular activity may not interfere with a detection of atrial sense events.

Generally, a detection for atrial sense events takes place outside of the blanking window T_{blank}. As it shall be explained further below with reference to Fig. 5, an atrial detection window Tₛₑₙₛₑ is started after lapse of the blanking window T_{blank}.

An atrial sense event As is generally assumed to be present if, in the atrial detection window Tₛₑₙₛₑ, e.g. the signal S2 crosses a sense threshold ST, as it is shown in Fig. 4B. If an atrial sense event As is detected, as it is the case for the second cardiac cycle in Fig. 4B, the atrial sense event As is used to derive timing information for triggering a ventricular pace (if necessary).

Referring now to Fig. 5, in the timing stage 2 of the processing circuitry 15 a timing for a pacing action and in addition for placing the atrial detection window Tₛₑₙₛₑ in a cardiac cycle may be determined. The timing herein is based on the use of various timers, which cause timeouts, such that based on at least one of the timeouts a pacing action may be triggered.

It shall be noted that the timing stage 2, as subsequently described according to Figs. 5 to 7, may be implemented on a chip and hence may be hardwired in hardware, wherein it also is possible to implement a timer model as described below in software for execution within a processor of the processing circuitry 15.

The timing stage 2, as shown in one embodiment in Fig. 5, comprises a main timer module 20, which has the shape of a counter and uses, as input, a clock signal C based on which it is counted upwards starting from an initial reset state.

The main timer module 20 is reset, using a main timer reset command MTR, at the start of each cardiac cycle. In particular, at a ventricular event, which may be a ventricular sense event Vs due to intrinsic ventricular activity or a ventricular pace event Vp due to a pacing action causing a ventricular contraction, the main timer reset command MTR causes the main timer module 20 to be reset and hence to start counting at 0.

The main timer module 20 feeds its timer count value (representing a current timer value) to different comparator modules 21, 22, 23, which compare the current timer count value to specified parameters in order to determine the position of the atrial detection window Tₛₑₙₛₑ within the current cardiac cycle and to determine timeouts TO2, TO3 based on the output of the main timer module 20.

The count value of the main timer module 20, in particular, is fed to a comparator module 21 serving to trigger the start of the atrial detection window Tₛₑₙₛₑ. For this, the comparator module 21 uses as input the count value of the main timer module 20 and an atrial detection window start time AWS. Based on a comparison of the actual count value as provided by the main timer module 20 and the atrial detection window start time AWS a latch module 210 is set. Namely, once the count value as provided by the main timer module 20 has reached the atrial detection window start time AWS, the latch module 210 is set to a logical on state indicating the start of the atrial detection window Tₛₑₙₛₑ.

A inverted output Q of the latch module 210 is connected to a reset input R of a latch module 211, such that the latch module 211 is held in a reset state as long as the atrial detection window Tₛₑₙₛₑ has not yet started. Once the atrial detection window Tₛₑₙₛₑ has started, based on the logical on state of the latch module 210 the latch module 211 is no longer held reset, and a detection of an atrial event As, which is fed as an input to the latch module 211, causes the latch module 211 to switch to a logical on state.

The latch module 211 is connected, with its positive output Q, to an AND gate 213 and, with its inverted output Q, to an AND gate 212.

The AND gate 213 receives the clock signal C as a further input, such that the clock signal C is fed to a timer module 24 if the latch module 211 is in its logical on state, indicating hence that an atrial event As has been detected in the atrial detection window Tₛₑₙₛₑ. The timer module 24 hence counts upwards starting with the detection of the atrial event As in the atrial detection window Tₛₑₙₛₑ, feeding the count value to a comparator module 240, which compares the count value to a first pacing delay AVD, also denoted as atrioventricular delay or atrial-ventricular delay, and sets a latch module 241 to a logical on state if the count value matches the first pacing delay. In this case, at the positive output of the latch module 241, an AV timeout TO1 is signaled.

The AND gate 212, as a further input next to the inverted output Q of the latch module 211, is connected to a positive output of a latch module 220. The latch module 220 is connected to and set by a comparator module 22, which takes as inputs the count value of the main timer module 20 and a second pacing delay VVD, also denoted as ventricular-ventricular delay, such that the latch module 220 is set to a logical on state in case the count value as output by the main timer module 20 matches the second pacing delay VVD.

If the second pacing delay VVD is reached and hence the latch module 220 is set to its logical on state, and if in addition no atrial event As has (yet) been detected in the atrial detection window Tₛₑₙₛₑ, as indicated by the inverted output Q of the latch module 211, a so-called flywheel timeout TO2 is set by the AND gate 212, indicating that the second pacing delay VVD has elapsed and so far no atrial event As has been detected.

The count value as output by the main timer module 20 is furthermore fed to a comparator module 23, which compares the count value to a basic rate interval BRI. Once the basic rate interval BRI is reached, indicating a longest interval that the patient should experience without either a ventricular sense or pace and is programmed for example by a clinician, a latch module 230 is switched to a logical on state, and a third timeout TO3, a so-called basic rate timeout, is set.

The count value of the main timer module 20 furthermore is fed to a latch module 25, which outputs the count value in case a ventricular sense event Vs or a ventricular pace event Vp, as combined by an OR gate 250, is present. The latch module 25 hence outputs a value for the interval between a prior ventricular event and a now occurring, subsequent ventricular event as measured for the current cardiac cycle (in Fig. 5: VVI = V-V interval).

The interval as output by the latch module 25 may be used to determine an average cardiac cycle interval time, for example by employing a moving averaging filter within which intervals as output by the latch module 25 are averaged using a moving window over a predefined number of cardiac cycles.

The average cardiac cycle interval time, in one embodiment, is used to set the start time AWS for the atrial detection window Tₛₑₙₛₑ, as input to the comparator module 21. In particular, the atrial detection window start time AWS may be set based on a lookup for example in a stored lookup table using the average cardiac cycle interval time.

In addition, also the length of the atrial detection window Tₛₑₙₛₑ may be determined based on a lookup for example in a stored lookup table using the average cardiac cycle interval time.

Further, the second pacing delay VVD is set based on the average cardiac cycle interval time. In particular, the second pacing delay VVD may be set to equal the average cardiac cycle interval time, such that the second pacing delay VVD indicates that time span after which, following a prior ventricular event, a ventricular sense event Vs is to be expected, or may be set based on a lookup using the average cardiac cycle interval time as input.

In addition, the first pacing delay AVD may be set based on the average cardiac cycle interval time, for example using a lookup in a stored lookup table.

The basic rate interval BRI for example is programmed by a clinician and hence is fixed. The basic rate interval BRI generally indicates a longest time span that the patient should experience without occurrence of a ventricular event Vp, Vs.

The atrial detection window start time AWS, the second pacing delay VVD, the basic rate interval BRI as well as the first pacing delay AVD are indicated in Fig. 4B.

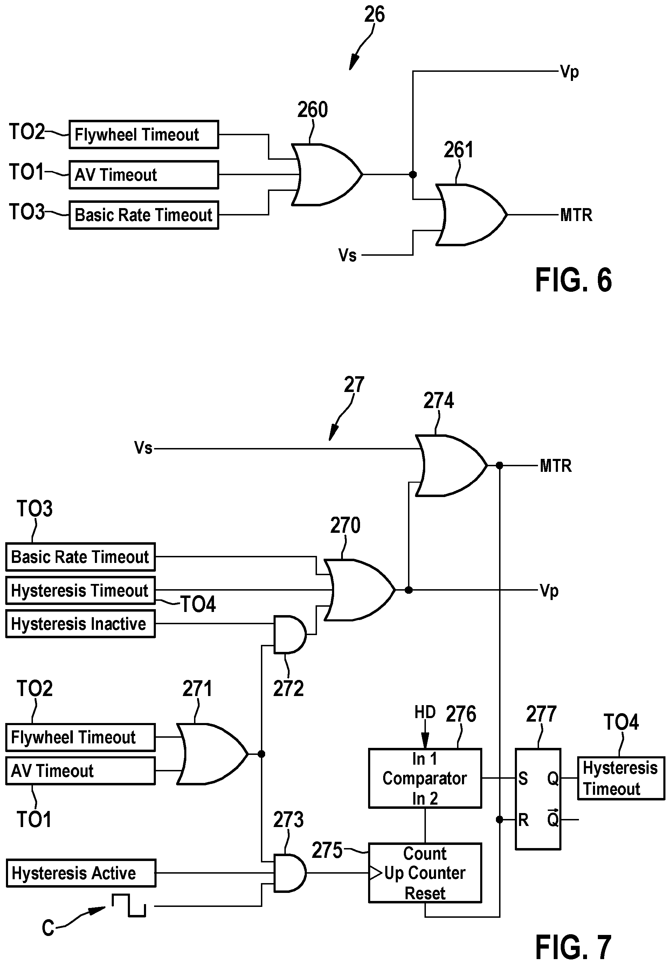

The different timeouts TO1, TO2, TO3, as identified using the schematic circuit of Fig. 5, are used as inputs to a combiner module 26, as illustrated in Fig. 6. Namely, the different timeouts TO1, TO2, TO3 are combined by an OR gate 260, which causes a ventricular pace Vp in case any one of the timeouts TO1, TO2, TO3 is identified, hence indicating that one of the first pacing delay AVD, the second pacing delay VVD and the basic rate interval BRI has elapsed without sensing an intrinsic ventricular sense event Vs.

If a ventricular pace Vp is done, or if a ventricular sense event Vs prior to latching any of the timeouts TO1, TO2, TO3 is detected, a main timer reset command MTR is triggered as output of an OR gate 261 in Fig. 6.

Referring now again to Fig. 5, the main timer reset command MTR causes the main timer module 20 as well as the timer module 24 and the latch modules 210, 220, 230, 241 to be reset. The timer modules 20, 24 hence start counting anew at 0, and the latch modules 210, 220, 230, 241 are set to their logical off state.

In the embodiment of Fig. 6, a pace event Vp to do a ventricular pace is triggered as soon as one of the timeouts TO1, TO2, TO3 is detected and hence one of the delays AVD, VVD, BRI has elapsed. This corresponds to a mode of operation of the processing circuitry 15 without a hysteresis, hence without an additional delay after lapse of the first pacing delay AVD and the second pacing delay VVD.

Referring now to Fig. 7, in a combiner module 27 implementing a hysteresis mode a ventricular pace Vp is triggered, in case a hysteresis state is set to active, if an additional hysteresis delay HD has elapsed after detection of any one of the timeouts TO1, TO2 representing a lapse of the first pacing delay AVD and the second pacing delay VVD.

Specifically, the timeouts TO1, TO2 are combined using an OR gate 271 whose output is fed to AND gates 272, 273.

The AND gate 273 herein obtains as input the hysteresis active state (indicating that hysteresis shall be used) and the clock signal C, and the output of the AND gate 273 is fed to a timer module 275 counting up from the time that any one of the timeouts TO1, TO2 is detected. The current value of the timer module 275 is fed to a comparator module 276, which compares the count value to the hysteresis delay HD and sets a latch module 277 to a logical on state in case the count value as output by the timer module 275 matches the hysteresis delay HD. If hence the hysteresis delay HD is found to have elapsed, the latch module 277 sets a hysteresis timeout TO4.

Signals indicating the hysteresis timeout TO4 and the basic rate timeout TO3 are fed to an OR gate 270, in addition to the output of the AND gate 272 combining the output of the OR gate 271 and a hysteresis inactive state (indicating that hysteresis shall not be used).

If hysteresis is inactive and hence no hysteresis shall be used, thus the basic rate timeout TO3 together with the AV timeout TO1 and the flywheel timeout TO2 are fed to the OR gate 270, causing a ventricular pace Vp if any one of the timeouts TO1, TO2, TO3 is detected. If hysteresis is inactive, hence, the combiner module 27 behaves like the combiner module 26 of Fig. 6.

In contrast, if hysteresis shall be used and hence the hysteresis active state is set, the basic rate timeout TO3 together with the hysteresis timeout TO4 as set by the latch module 277 is fed to the OR gate 270, the hysteresis timeout TO4 indicating that any one of the flywheel timeout TO2 and the AV timeout TO1 plus the additional hysteresis delay HD have elapsed. Accordingly, if any one of the basic rate timeout TO3 and the hysteresis timeout TO4 is present, a ventricular pace Vp is caused.

If a ventricular pace Vp is caused, or if a ventricular sense event Vs is detected prior to the lapse of any of the timeouts TO1 to TO4, an OR gate 274 sets the main timer reset command MTR, thus resetting the circuitry of Fig. 5 as well as the latch module 277 and the timer module 275 in the combiner module 27 of Fig. 7.

The hysteresis active state may be set, in the embodiment of Fig. 7, based on a detection of prior detection of atrial ventricular conduction events. Generally, if recently atrial ventricular conduction events have been detected, it may be assumed that intrinsic ventricular activity is likely to occur, such that it should be given an additional waiting time after occurrence of the timeout TO1 or the timeout TO2 to give preference to intrinsic conduction over a ventricular pacing. The hysteresis active state may for example be assessed based on a logic that compares the ratio of ventricular senses to ventricular paces, for example by employing an up/down counter which counts up for each ventricular sense and counts down for each ventricular pace. By comparing a count value of the up/down counter for example to a threshold the hysteresis active state may be set, or alternatively a hysteresis inactive state may be set such that no hysteresis mode is employed.

The timing stage 2 as illustrated in Fig. 5 determines different timeouts based on various delays, which at least in part are set based on the average cardiac cycle interval time. Herein, a reliable pacing depends on whether ventricular sense events Vs and/or atrial sense events As can be reliably detected and hence the average cardiac cycle interval time for successive cardiac cycles can be determined.

In one embodiment, the timing stage 2 as illustrated in Fig. 5 is based on a processing of a cardiac sense signal of a sensing arrangement, such as an arrangement of the electrodes 11 and 12, hereinafter also referred to as electrode arrangement 11, 12, as described previously with reference to Fig. 2. Herein, as illustrated in Fig. 2, the leadless implantable medical device 1 may comprise another sensing arrangement 19, which may employ a different sensing technology in comparison to the electrode arrangement 11, 12. For example, the sensing arrangement 19 may be a motion sensor, for example an accelerometer, for detecting motion signals. Alternatively, the sensing arrangement 19 may be a pressure sensor, a flow sensor, an acoustic sensor, an ultrasound sensor, an impedance sensor or the like. The sensing arrangement 19 may also be called sensing device 19. The electrode arrangement 11, 12 may also be called sensing arrangement 11, 12.

The additional sensing arrangement 19 may be used as an alternate source in case it is found that the average cardiac cycle interval time cannot be reliably determined based on processing cardiac sense signals as output e.g. by the electrode arrangement 11, 12.

For example, when it is found that no ventricular sense events Vs and/or atrial sense events As are detected for a predefined number of cardiac cycles based on a processing of a sense signal as output by the electrode arrangement 11, 12, it may be turned to an alternate source, e.g. a sensing signal of the sensing arrangement 19, to determine the average cardiac cycle interval time. In particular, if the number of cardiac cycles for which no ventricular sense events Vs and/or atrial sense events As are identified exceeds a predefined threshold, processing is switched to an alternate source, e.g. a sensing signal of the sensing arrangement 19 in order to determine the average cardiac cycle interval time.

As another alternate source, a pre-programmed resting rate may be used to set the average cardiac cycle interval time if the average cardiac cycle interval time cannot be determined based on the electrode arrangement 11, 12 and possibly also not based on the additional sensing arrangement 19. Hence, if the average cardiac cycle interval time cannot be set based on ventricular events Vs as sensed based on a processing of cardiac sense signals, it may be turned to a preprogrammed resting rate, such that a preprogrammed value is used for the average cardiac cycle interval time for setting the start time AWS of the atrial detection window Tₛₑₙₛₑ, the first pacing delay AVD and the second pacing delay VVD.

Rather than switching immediately to a value for the average cardiac cycle interval time as derived based on the alternate source, in one embodiment a rate change of the average cardiac cycle interval time may be controlled using a logic, such that a smooth transition for the value of the average cardiac cycle interval time average cardiac cycle interval time is ensured when switching from a processing of sense signals to an alternate source.

### List of reference numerals

- 1: Leadless implantable medical device (leadless pacemaker device)
- 10: Body (housing)
- 100: Tip
- 101: End
- 11: First electrode (pacing electrode)
- 12: Second electrode
- 14: Fixation device
- 15: Processing circuitry
- 16: First processing channel
- 161: Amplification stage
- 162: Detection stage
- 17: Second processing channel
- 171: Amplification stage
- 172: Windowing stage
- 173: Detection stage
- 18: Battery
- 19: Sensing device
- 2: Timing stage
- 20: Main timer
- 21: Comparator module
- 210, 211: Latch module
- 212, 213: AND gate
- 22: Comparator module
- 220: Latch module
- 23: Comparator module
- 230: Latch module
- 24: AV timer
- 240: Comparator module
- 241: Latch module
- 25: Latch module
- 250: OR gate
- 26: Combiner module
- 260, 261: OR gate
- 27: Combiner module
- 270,271: OR gate
- 272, 273: AND gate
- 274: OR gate
- 275: Hysteresis timer
- 276: Comparator module
- 277: Latch module
- As: Atrial sense event
- AVD: First pacing delay
- AVN: Atrioventricular node
- AWS: Start time
- BRI: Basic rate interval
- C: Clock
- G1, G2: Gain
- Hi: HIS bundle
- HD: Hysteresis delay
- LA: Left atrium
- LV: Left ventricle
- M: Intra-cardiac tissue (myocardium)
- MTR: Main timer reset command
- RA: Right atrium
- RV: Right ventricle
- S1, S2: Signal
- SAN: Sinoatrial node
- ST: Sense threshold
- T_{blank}: Blanking window
- Tₛₑₙₛₑ: Detection window
- TO1-TO4: Timeout
- V: Ventricular vector
- Vp: Ventricular pace
- Vs: Ventricular sense event
- VVD: Second pacing delay (flywheel interval)

## Claims

1. An implantable medical device (1) configured to provide for an intra-cardiac pacing, the implantable medical device (1) comprising:
an electrode arrangement (11, 12) configured to sense a cardiac sense signal; and
a processing circuitry (15) operatively connected to the electrode arrangement (11, 12), wherein the processing circuitry (15) is configured
to start a first timer (20) based on a ventricular sense event (Vs) identified using the cardiac sense signal or based on a ventricular pace event (Vp);
to open an atrial detection window (Tₛₑₙₛₑ) based on a first timer count output by the first timer (20);
to start a second timer (24) in case an atrial sense event (As) is identified using the cardiac sense signal after opening the atrial detection window (Tₛₑₙₛₑ);
to identify a first timeout (TO1) based on a comparison of a second timer count output by the second timer (24) and a first pacing delay (AVD) indicative of a delay after which a ventricular pace signal should be triggered following a prior atrial sense event (As); **characterised in that** the processing circuitry is further configured
to identify a second timeout (TO2) based on a comparison of the first timer count output by the first timer (20) and a second pacing delay (VVD) indicative of a delay after which a ventricular pace signal should be triggered following a prior ventricular sense event (Vs) or ventricular pace event (Vp);
to identify a third timeout (TO3) based on a comparison of the first timer count output by the first timer (20) and a basic rate interval (BRI) indicative of a longest allowable interval without a ventricular sense event (Vs) or ventricular pace event (Vp); and
to trigger a ventricular pace signal based on an identification of at least one of the first timeout (TO1), the second timeout (TO2) and the third timeout (TO3).

2. The implantable medical device (1) according to claim 1, wherein the processing circuitry (15) is configured to open the atrial detection window (Tₛₑₙₛₑ) based on a comparison of said first timer count output by the first timer (20) to an atrial detection window start time (AWS).

3. The implantable medical device (1) according to claim 2, wherein the processing circuitry (15) is configured to set said atrial detection window start time (AWS) based on an average cardiac cycle interval time.

4. The implantable medical device (1) according to one of claims 1 to 3, wherein the processing circuitry (15) is configured to set said first pacing delay (AVD) based on an average cardiac cycle interval time.

5. The implantable medical device (1) according to one of the preceding claims, wherein the processing circuitry (15) is configured to set said second pacing delay (VVD) based on an average cardiac cycle interval time.

6. The implantable medical device (1) according to one claims 3 to 5, wherein the processing circuitry (15) is configured to determine a ventricular-ventricular interval based on said first timer count at the time of a ventricular sense event (Vs) or a ventricular pace event (Vp) and to compute said average cardiac cycle interval time based on the ventricular-ventricular interval.

7. The implantable medical device (1) according to claim 6, wherein the processing circuitry (15) is configured to determine the ventricular-ventricular interval based on a sense signal received with a sensing device (19) other than said electrode arrangement (11, 12) in case no ventricular sense event (Vs) is identified in a predefined number of cardiac cycles.

8. The implantable medical device (1) according to one of the preceding claims, wherein the processing circuitry (15) is configured to identify said second timeout (TO2) if said first timer count matches the second pacing delay (VVD) and no atrial sense event (As) is identified after opening the atrial detection window (Tₛₑₙₛₑ).

9. The implantable medical device (1) according to one of the preceding claims, wherein the processing circuitry (15) is configured to reset the first timer (20) and the second timer (24) in case a ventricular sense event (Vs) is identified based on the cardiac sense signal or in case a ventricular pace event (Vp) is triggered.

10. The implantable medical device (1) according to one of the preceding claims, wherein the processing circuitry (15) is configured to start a third timer (276) based on the identification of at least one of the first timeout (TO1) and the second timeout (TO2) and to identify a fourth timeout (TO4) based on a comparison of a third timer count output by the third timer (276) and a hysteresis delay (HD) indicative of a delay after said first timeout (TO1) or said second timeout (TO2).

11. The implantable medical device (1) according to claim 10, wherein the processing circuitry (15) is configured to trigger a ventricular pace signal based on the identification of the fourth timeout (TO4).

12. The implantable medical device (1) according to claim 10 or 11, wherein the processing circuitry (15) is configured to set said hysteresis delay (HD) based on an average cardiac cycle interval time.

13. The implantable medical device (1) according to one of claims 10 to 12, wherein the processing circuitry (15) is configured to set a hysteresis active signal based on at least one prior ventricular sense event (Vs) and to start said third timer (276) only if the hysteresis active signal is set.

14. The implantable medical device (1) according to one of claims 10 to 13, wherein the processing circuitry (15) is configured to reset the third timer (276) in case a ventricular sense event (Vs) is identified based on the cardiac sense signal or in case a ventricular pace event (Vp) is triggered.

## Patentansprüche

1. Implantierbare medizinische Vorrichtung (1), die ausgebildet ist, eine intrakardiale Schrittgebung bereitzustellen, wobei die implantierbare medizinische Vorrichtung (1) umfasst:
eine Elektrodenanordnung (11, 12), die ausgebildet ist, ein kardiales Erfassungssignal zu erfassen; und
eine Verarbeitungsschaltung (15), die wirkmäßig mit der Elektrodenanordnung (11, 12) verbunden ist, wobei die Verarbeitungsschaltung (15) ausgebildet ist zum
Starten eines ersten Zeitmessers (20) auf Basis eines ventrikulären Erfassungsereignisses (Vs), das unter Verwendung des kardialen Erfassungssignals oder auf Basis eines ventrikulären Schrittgebungsereignisses (Vp) identifiziert wird;
Öffnen eines atrialen Detektionsfensters (Tₛₑₙₛₑ) auf Basis eines ersten Zeitmesserzählwerts, der von dem ersten Zeitmesser (20) ausgegeben wird;
Starten eines zweiten Zeitmessers (24), falls ein atriales Erfassungsereignis (As) unter Verwendung des kardialen Erfassungssignals identifiziert wird, nachdem das atriale Detektionsfenster (Tₛₑₙₛₑ) geöffnet wurde;
Identifizieren einer ersten Zeitüberschreitung (TO1) auf Basis eines Vergleichs eines zweiten Zeitmesserzählwerts, der von dem zweiten Zeitmesser (24) ausgegeben wird, und einer ersten Schrittgebungsverzögerung (AVD), die eine Verzögerung angibt, nach der ein ventrikuläres Schrittgebungssignal als Folge eines vorangehenden atrialen Erfassungsereignisses (As) ausgelöst werden sollte, **dadurch gekennzeichnet, dass** die Verarbeitungsschaltung ferner ausgebildet ist zum
Identifizieren einer zweiten Zeitüberschreitung (TO2) auf Basis eines Vergleichs des ersten Zeitmesserzählwerts, der von dem ersten Zeitmesser (20) ausgegeben wird, und einer zweiten Schrittgebungsverzögerung (VVD), die eine Verzögerung angibt, nach der ein ventrikuläres Schrittgebungssignal als Folge eines vorangehenden ventrikulären Erfassungsereignisses (Vs) oder ventrikulären Schrittgebungsereignisses (Vp) ausgelöst werden sollte;
Identifizieren einer dritten Zeitüberschreitung (TO3) auf Basis eines Vergleichs des ersten Zeitmesserzählwerts, der von dem ersten Zeitmesser (20) ausgegeben wird, und eines Basisratenintervalls (BRI), das ein längstes zulässiges Intervall ohne ein ventrikuläres Erfassungsereignis (Vs) oder ventrikuläres Schrittgebungsereignis (Vp) angibt; und
Auslösen eines ventrikulären Schrittgebungssignals auf Basis einer Identifizierung von mindestens einer von der ersten Zeitüberschreitung (TO1), der zweiten Zeitüberschreitung (TO2) und der dritten Zeitüberschreitung (TO3).

2. Implantierbare medizinische Vorrichtung (1) nach Anspruch 1, wobei die Verarbeitungsschaltung (15) ausgebildet ist, das atriale Detektionsfenster (Tₛₑₙₛₑ) auf Basis eines Vergleichs des ersten Zeitmesserzählwerts, der vom ersten Zeitmesser (20) ausgegeben wird, mit einer Startzeit des atrialen Detektionsfensters (AWS) zu öffnen.

3. Implantierbare medizinische Vorrichtung (1) nach Anspruch 2, wobei die Verarbeitungsschaltung (15) ausgebildet ist, die Startzeit des atrialen Detektionsfensters (AWS) auf Basis einer durchschnittlichen Herzzyklusintervallzeit einzustellen.

4. Implantierbare medizinische Vorrichtung (1) nach einem der Ansprüche 1 bis 3, wobei die Verarbeitungsschaltung (15) ausgebildet ist, die erste Schrittgebungsverzögerung (AVD) auf Basis einer durchschnittlichen Herzzyklusintervallzeit einzustellen.

5. Implantierbare medizinische Vorrichtung (1) nach einem der vorangehenden Ansprüche, wobei die Verarbeitungsschaltung (15) ausgebildet ist, die zweite Schrittgebungsverzögerung (VVD) auf Basis einer durchschnittlichen Herzzyklusintervallzeit einzustellen.

6. Implantierbare medizinische Vorrichtung (1) nach einem der Ansprüche 3 bis 5, wobei die Verarbeitungsschaltung (15) ausgebildet ist, ein ventrikulär-ventrikuläres Intervall auf Basis des ersten Zeitmesserzählwerts zur Zeit eines ventrikulären Erfassungsereignisses (Vs) oder eines ventrikulären Schrittgebungsereignisses (Vp) zu bestimmen und die durchschnittliche Herzzyklusintervallzeit auf Basis des ventrikulär-ventrikulären Intervalls zu berechnen.

7. Implantierbare medizinische Vorrichtung (1) nach Anspruch 6, wobei die Verarbeitungsschaltung (15) ausgebildet ist, das ventrikulär-ventrikuläre Intervall auf Basis eines Erfassungssignals zu bestimmen, das mit einer Erfassungsvorrichtung (19) empfangen wird, bei der es sich nicht um die Elektrodenanordnung (11, 12) handelt, falls in einer vordefinierten Anzahl von Herzzyklen kein ventrikuläres Erfassungsereignis (Vs) identifiziert wird.

8. Implantierbare medizinische Vorrichtung (1) nach einem der vorangehenden Ansprüche, wobei die Verarbeitungsschaltung (15) ausgebildet ist, die zweite Zeitüberschreitung (TO2) zu identifizieren, wenn der erste Zeitmesserzählwert mit der zweiten Schrittgebungsverzögerung (VVD) übereinstimmt und kein atriales Erfassungsereignis (As) identifiziert wird, nachdem das atriale Detektionsfenster (Tₛₑₙₛₑ) geöffnet wurde.

9. Implantierbare medizinische Vorrichtung (1) nach einem der vorangehenden Ansprüche, wobei die Verarbeitungsschaltung (15) ausgebildet ist, den ersten Zeitmesser (20) und den zweiten Zeitmesser (24) zurückzusetzen, falls ein ventrikuläres Erfassungsereignis (Vs) auf Basis des kardialen Erfassungssignals identifiziert wird oder falls ein ventrikuläres Schrittgebungsereignis (Vp) ausgelöst wird.

10. Implantierbare medizinische Vorrichtung (1) nach einem der vorangehenden Ansprüche, wobei die Verarbeitungsschaltung (15) ausgebildet ist, einen dritten Zeitmesser (276) auf Basis der Identifizierung von mindestens einer von der ersten Zeitüberschreitung (TO1) und der zweiten Zeitüberschreitung (TO2) zu starten und auf Basis eines Vergleichs eines dritten Zeitmesserzählwerts, der vom dritten Zeitmesser (276) ausgegeben wird, und einer Hystereseverzögerung (HD), die eine Verzögerung nach der ersten Zeitüberschreitung (TO1) oder der zweiten Zeitüberschreitung (TO2) angibt, eine vierte Zeitüberschreitung (TO4) zu identifizieren.

11. Implantierbare medizinische Vorrichtung (1) nach Anspruch 10, wobei die Verarbeitungsschaltung (15) ausgebildet ist, ein ventrikuläres Schrittgebungssignal auf Basis der Identifizierung der vierten Zeitüberschreitung (TO4) auszulösen.

12. Implantierbare medizinische Vorrichtung (1) nach Anspruch 10 oder 11, wobei die Verarbeitungsschaltung (15) ausgebildet ist, die Hystereseverzögerung (HD) auf Basis einer durchschnittlichen Herzzyklusintervallzeit einzustellen.

13. Implantierbare medizinische Vorrichtung (1) nach einem der Ansprüche 10 bis 12, wobei die Verarbeitungsschaltung (15) ausgebildet ist, ein Hysterese-aktiv-Signal auf Basis mindestens eines früheren ventrikulären Erfassungsereignisses (Vs) zu setzen und den dritten Zeitmesser (276) nur dann zu starten, wenn das Hysterese-aktiv-Signal gesetzt ist.

14. Implantierbare medizinische Vorrichtung (1) nach einem der Ansprüche 10 bis 13, wobei die Verarbeitungsschaltung (15) ausgebildet ist, den dritten Zeitmesser (276) zurückzusetzen, falls auf Basis des kardialen Erfassungssignals ein ventrikuläres Erfassungsereignis (Vs) identifiziert wird oder falls ein ventrikuläres Schrittgebungsereignis (Vp) ausgelöst wird.

## Revendications

1. Dispositif médical implantable (1) conçu pour pourvoir à une stimulation intracardiaque, le dispositif médical implantable (1) comprenant :
un agencement d'électrodes (11, 12) conçu pour détecter un signal de détection cardiaque ; et un circuit de traitement (15) connecté de manière fonctionnelle à l'agencement d'électrodes (11, 12), dans lequel le circuit de traitement (15) est conçu
pour démarrer un premier chronomètre (20) sur la base d'un événement de détection ventriculaire (Vs) identifié en utilisant le signal de détection cardiaque ou sur la base d'un événement de stimulation ventriculaire (Vp) ;
pour ouvrir une fenêtre de détection auriculaire (T_{détection}) sur la base d'un renvoi de décompte de premier chronomètre par le premier chronomètre (20) ;
pour démarrer un deuxième chronomètre (24) dans le cas où un événement de détection auriculaire (As) est identifié en utilisant le signal de détection cardiaque après ouverture de la fenêtre de détection auriculaire (T_{détection}) ;
pour identifier un premier arrêt (TO1) sur la base d'une comparaison d'un renvoi de décompte du deuxième chronomètre par le deuxième chronomètre (24) et d'un premier délai de stimulation (AVD) indiquant un délai après lequel un signal de stimulation ventriculaire devrait être déclenché après un événement de détection auriculaire (As) antérieur ; **caractérisé en ce que** le circuit de traitement est en outre conçu
pour identifier un deuxième arrêt (TO2) sur la base d'une comparaison du renvoi de décompte de premier chronomètre par le premier chronomètre (20) et d'un second délai de stimulation (VVD) indiquant un délai après lequel un signal de stimulation ventriculaire devrait être déclenché après un événement de détection ventriculaire (Vs) ou un événement de stimulation ventriculaire (Vp) antérieur ;
pour identifier un troisième arrêt (TO3) sur la base d'une comparaison du renvoi de décompte de premier chronomètre par le premier chronomètre (20) et d'un intervalle de fréquence de base (BRI) indiquant le plus long intervalle acceptable sans événement de détection ventriculaire (Vs) ou événement de stimulation ventriculaire (Vp) ; et
pour déclencher un signal de stimulation ventriculaire sur la base d'une identification d'au moins un parmi le premier arrêt (TO1), le deuxième arrêt (TO2) et le troisième arrêt (TO3).

2. Dispositif médical implantable (1) selon la revendication 1, dans lequel le circuit de traitement (15) est conçu pour ouvrir la fenêtre de détection auriculaire (T_{détection}) sur la base d'une comparaison dudit renvoi de décompte de premier chronomètre par le premier chronomètre (20) à un temps de départ de fenêtre de détection auriculaire (AWS).

3. Dispositif médical implantable (1) selon la revendication 2, dans lequel le circuit de traitement (15) est conçu pour paramétrer ledit temps de départ de fenêtre de détection auriculaire (AWS) sur la base d'un temps d'intervalle de cycle cardiaque moyen.

4. Dispositif médical implantable (1) selon l'une des revendications 1 à 3, dans lequel le circuit de traitement (15) est conçu pour paramétrer ledit premier délai de stimulation (AVD) sur la base d'un temps d'intervalle de cycle cardiaque moyen.

5. Dispositif médical implantable (1) selon l'une des revendications précédentes, dans lequel le circuit de traitement (15) est conçu pour paramétrer ledit second délai de stimulation (VVD) sur la base d'un temps d'intervalle de cycle cardiaque moyen.

6. Dispositif médical implantable (1) selon l'une des revendications 3 à 5, dans lequel le circuit de traitement (15) est conçu pour déterminer un intervalle ventriculaire-ventriculaire sur la base dudit décompte de premier chronomètre au temps d'un événement de détection ventriculaire (Vs) ou d'un événement de stimulation ventriculaire (Vp) et pour calculer ledit temps d'intervalle de cycle cardiaque moyen sur la base de l'intervalle ventriculaire-ventriculaire.

7. Dispositif médical implantable (1) selon la revendication 6, dans lequel le circuit de traitement (15) est conçu pour déterminer l'intervalle ventriculaire-ventriculaire sur la base d'un signal de détection reçu avec un dispositif de détection (19) différent dudit agencement d'électrodes (11, 12) dans le cas où aucun événement de détection ventriculaire (Vs) n'est identifié dans un nombre prédéfini de cycles cardiaques.

8. Dispositif médical implantable (1) selon l'une des revendications précédentes, dans lequel le circuit de traitement (15) est conçu pour identifier ledit deuxième arrêt (TO2) si ledit décompte de premier chronomètre correspond au second délai de stimulation (VVD) et qu'aucun événement de détection auriculaire (As) n'est identifié après l'ouverture de la fenêtre de détection auriculaire (T_{détection}).

9. Dispositif médical implantable (1) selon l'une des revendications précédentes, dans lequel le circuit de traitement (15) est conçu pour réinitialiser le premier chronomètre (20) et le deuxième chronomètre (24) dans le cas où un événement de détection ventriculaire (Vs) est identifié sur la base du signal de détection cardiaque ou dans le cas où un événement de stimulation ventriculaire (Vp) est déclenché.

10. Dispositif médical implantable (1) selon l'une des revendications précédentes, dans lequel
le circuit de traitement (15) est conçu pour démarrer un troisième chronomètre (276) sur la base de l'identification d'au moins un parmi le premier arrêt (TO1) et le deuxième arrêt (TO2) et pour identifier un quatrième arrêt (TO4) sur la base d'une comparaison d'un renvoi de décompte de troisième chronomètre par le troisième chronomètre (276) et d'un délai d'hystérésis (HD) indiquant un délai après ledit premier arrêt (TO1) ou ledit deuxième arrêt (TO2).

11. Dispositif médical implantable (1) selon la revendication 10, dans lequel le circuit de traitement (15) est conçu pour déclencher un signal de stimulation ventriculaire sur la base de l'identification du quatrième arrêt (TO4).

12. Dispositif médical implantable (1) selon la revendication 10 ou 11, dans lequel le circuit de traitement (15) est conçu pour paramétrer ledit délai d'hystérésis (HD) sur la base d'un temps d'intervalle de cycle cardiaque moyen.

13. Dispositif médical implantable (1) selon l'une des revendications 10 à 12, dans lequel le circuit de traitement (15) est conçu pour paramétrer un signal actif d'hystérésis sur la base d'au moins un événement de détection ventriculaire (Vs) antérieur et pour démarrer ledit troisième chronomètre (276) uniquement si le signal actif d'hystérésis est paramétré.

14. Dispositif médical implantable (1) selon l'une des revendications 10 à 13, dans lequel le circuit de traitement (15) est conçu pour réinitialiser le troisième chronomètre (276) dans le cas où un événement de détection ventriculaire (Vs) est identifié sur la base du signal de détection cardiaque ou dans le cas où un événement de stimulation ventriculaire (Vp) est déclenché.
